# EUROPEAN PATENT APPLICATION

(11) **EP 4 089 112 A1**
(43) Date of publication of application: **16.11.2022**
(21) Application number: 21173971.9
(22) Date of filing: 14.05.2021
(51) Int. Cl.: C07K 16/10, A61P 31/14, G01N 33/577

(54) **ANTIBODY BINDING TO RBD OF SPIKE PROTEIN OF SARS-COV-2 AND A METHOD FOR QUANTIFYING PROTECTIVE ANTIBODIES AGAINST SARS-COV-2**

(71) Applicant: Ustav organicke chemie a biochemie AV CR, v.v.i., 16610 Praha (CZ); Biotechnologicky ustav AV CR, v.v.i., 25250 Vestec (CZ)
(72) Inventor: Sacha, Pavel, 16300 Praha 17 (CZ); Weber, Jan, 26701 Králuv Dvur (CZ); Hodek, Jan, 17000 Praha 7 (CZ); Sedlak, Frantisek, 14000 Praha 4 (CZ); Moos, Jiri, 13000 Praha 3 (CZ); Plicka, Jan, 41201 Litomerice (CZ); Barinka, Cyril, 19016 Praha 9 (CZ); Novakova, Zora, 14900 Praha 4 (CZ)
(74) Representative: Hartvichova, Katerina

(57) **Abstract**

The present invention relates to a novel antibody binding to the RBD of the Spike protein of the SARS-CoV-2. The antibody recognizes a conformational epitope of the Spike RBD protein, inhibits Spike RBD - ACE2 binding, and inhibits viral infection in Virus Neutralization Test (VNT). The inhibition of the viral infection was observed for the wild-type SARS Cov-2 (variant B. 1.) as well as for the variant of concern (B. 1.1.7.) of the SARS CoV-2.

The invention further provides the method for detecting and quantifying protective antibodies against the SARS Cov-2 Spike protein using the novel antibody.

## Description

### Field of the Invention

The invention relates to a novel in vitro diagnostics tool in the field of SARS CoV-2 serology-based immunoassays to detect neutralizing (protective) antibodies in patient sample. More specifically, the invention relates to a competitive ELISA assay based on a monoclonal IgG antibody with unique neutralizing properties that inhibits SARS CoV-2 binding to the host cell ACE2 receptor.

### Background Art

Viral infection by severe acute respiratory syndrome coronavirus 2 (SARS CoV-2) is a cause of the infectious disease COVID-19. The infection induces an immune response producing antibodies against viral proteins. The SARS CoV-2 belongs to RNA viruses and is composed of four major structural and functional proteins. The first protein is the S protein (or Spike protein), a trimeric transmembrane glycoprotein that is exposed on the virus surface and mediates viral entry into host cells. The S protein consists of two functional subunits, S1 and S2. The S1 subunit contains the Receptor Binding Domain (RBD) responsible for the viral binding to host cells via angiotensin-converting enzyme 2 (ACE2) receptor, while the S2 domain mediates membrane fusion following Spike cleavage. The presence of antibodies against the S protein and its subunits can be detected within 1-3 weeks after the infection *(*Qu J, Wu C, Li X, Zhang G, Jiang Z, Li X, et al., Clin Infect Dis. 2020 Nov 19;71(16):2255-8, 2*;* Wolfel R, Corman VM, Guggemos W, Seilmaier M, Zange S, Muller MA, et al., Nature. 2020 May;581(7809):465-9*.).* The antibodies that can bind to the viral RBD and successfully block virus binding to the ACE2 at the plasma membrane of susceptible host cells are considered as neutralizing antibodies. The other major viral proteins are an abundantly expressed structural M protein (Membrane protein) and the N protein (Nucleocapsid), which can be detected in serum and urine samples during the first two weeks post-infection.
Serologic tests can identify individuals with a history of SARS CoV-2 infection or following a successful immunization. Thus, they can help to better understand the epidemiology of SARS -CoV-2 infection and to distinguish people at higher risk of infection. Although the immune response and antibody protection are not fully understood, the production of anti-SARS CoV-2 antibodies following SARS CoV-2 infection likely provides some degree of immunity from the subsequent infection for at least six months.
Several serological immunoassays have been developed to detect antibodies against SARS CoV-2 in the serum or plasma of patients, particularly IgM, IgG, and IgA against S and N proteins. The IgM plays the role of first defense against the viral infection. The IgM response to SARS CoV-2 was also reported approximately ten days after infection, but after 35 days, it rapidly declines and disappears. On the contrary, IgG provides long-term immunity and immunological memory. The emergence of IgG subtype antibodies is detectable starting from approximately two to three weeks post-infection, and they last for a longer time - a rough estimate is at least 4-6 months. IgA levels can be detected in the blood but also in the mucous membranes. IgA is essential for mucosal immunity and correlates with the severity of COVID-19 (Front. Bioeng. Biotechnol., 28 January 2021, https://doi.org/10.3389lfbioe.2020.605702).

Various types of assays are used to determine the immune response to SARS CoV-2 infection and the level and functionality of antibodies. The tests aim to detect either total binding antibodies or neutralizing/protecting antibodies.
Regarding the total binding antibody detection tests, more than 74 SARS CoV-2 serology/antibody diagnostic assays and other adaptive immune response tests have been developed and authorized by Food and Drug Administration (FDA) to date. Only 12 of those diagnostic assays are semi-quantitative.
The currently used serology-based assays include rapid lateral flow assays, enzyme-linked serological immunoassay (ELISA), chemiluminescent immunoassay (CIA), and automated chemiluminescent microparticle immunoassay (CMIA).
The lateral flow diagnostic method determines the qualitative presence (positive or negative) of antibodies in a blood specimen. This test is easy, provides results within minutes, and is cost-efficient. The lateral flow assays detect different isotypes of antibodies that target various SARS CoV-2 proteins (N and S proteins, among others).
Enzyme-linked serology-based immunoassays use purified SARS CoV-2 proteins to capture all binding antibodies from serum, plasma, whole blood, or dried blood spots. Individual antibody types - IgG, IgM, and IgA, are usually determined. Most of the currently used tests detect antibodies against either N or S proteins or both immunodominant proteins (multiplex assays). Unclear correlation of total binding antibody tests with the level of neutralizing antibodies remains a significant disadvantage.
ELISA is a conventional platform for SARS CoV-2 exposure assessment. It is a sensitive and specific method that can detect antibodies at the picogram/ml concentrations. It is generally set in microwell plates to detect and quantify antibodies produced against viral infection. The measured signal intensity reflects the total amount of binding antibodies present in the tested sample. The whole process usually takes 2-5 hours and can be semi-automated.

Neutralizing antibody detection tests may require up to 5 days to complete and additional lab biosafety level demands.
Those tests are used to assess the functional ability of antibodies to prevent virus infection - protectability. The tests monitor inhibition of viral growth in cell culture when incubated with patients' serum or plasma.
Virus neutralization tests (VNT) use a SARS CoV-2 virus from a clinical isolate or a recombinant SARS CoV-2 to cultivate the virus with patients' antibodies in vitro. Pseudovirus neutralization tests (pVNT) use recombinant pseudoviruses that display the S protein of the SARS CoV-2. For both the VNT and pVNT, completing the test usually takes several days to obtain the result, and those tests are thus not suitable for routine testing. Competitive neutralization tests (cVNT) are binding antibody tests designed to qualitatively detect potentially neutralizing antibodies. The assay often mimics RBD interaction with ACE2 in an ELISA-like format (similar to RBD binding to ACE2 surface receptor). The disadvantage of those tests is the difficult quantitation of neutralizing antibodies required to assess the immune response to SARS CoV-2 infection or vaccination.

### Disclosure of the Invention

The invention aimed to develop a monoclonal antibody-based quantitative assessment tool for determining the amount of protective/neutralizing antibodies against SARS CoV-2 Spike protein. The developed assay should complement the existing total binding antibody tests and the Virus Neutralization Tests (VNT, pVNT).

The present invention relates in the first aspect to an antibody binding to a conformational epitope located on Receptor Binding Domain (RBD) of Spike protein of SARS-CoV-2, wherein the CDRs (complementarity determining regions) of the variable heavy chain region have sequences:
TSGMGVS (SEQ ID NO. 3),
HIYWDDDERYNPSLKS (SEQ ID NO. 4), and
RSLYDYDGWFAY (SEQ ID NO. 5);
   and the CDRs of the variable light chain region have sequences:
KASQNVDTTVA (SEQ ID NO. 6),
SASSRYS (SEQ ID NO. 7), and
QQYNNYPLT (SEQ ID NO. 8).

In a preferred embodiment, the variable heavy chain region has a sequence with at least 90% identity, preferably with at least 95% identity, even more preferably with at least 97% identity, with the sequence EVQLRESGPGILQPSQTLSLTCSFSGFSLSTSGMGVSWIRQPSGKGLEWLAHIYWDDD ERYNPSLKSRLTISKDVSSNOVFLKIISVDTADTATYYCARRSLYDYDGWFAYWGOG TLVTVSA (SEQ ID NO. 1), wherein the underlined portions corresponding to CDRs are conserved.

Preferably, the variable heavy chain (VHC) region has a sequence differing by no more than 5, and more preferably by no more than 3, amino acid substitutions from SEQ ID NO. 1, wherein the amino acid substitutions are not in the CDRs.

More preferably, the variable heavy chain region has the sequence SEQ ID NO. 1.

In a preferred embodiment, the variable light chain region (VLC) has a sequence with at least 90% identity, preferably with at least 95% identity, even more preferably with at least 97% identity, with the sequence DIVMTOSOKFMSTSVGDRVSVTCKASONVDTTVAWYOOKPGOSPKALIYSASSRYS GVPDRFTGSGSGTDFTLTISNVQSEDLADYFCQQYNNYPLTFGAGTKLEIKRA (SEQ ID NO. 2), wherein the underlined portions corresponding to CDRs are conserved.

Preferably, the variable light chain region has a sequence differing by no more than 5, and more preferably by no more than 3, amino acid substitutions from SEQ ID NO. 2, wherein the amino acid substitutions are not in the CDRs.

More preferably, the variable light chain region has the sequence SEQ ID NO. 2.

It is preferred that if any amino acid substitutions in the sequences of the variable heavy chain region or the variable light chain region are present, then the amino acid substitutions are conservative amino acid substitutions. A conservative amino acid substitution refers to the replacement of an amino acid by another amino acid of the same nature, e.g., a hydrophobic amino acid is replaced by another hydrophobic amino acid, or a basic amino acid is replaced by another basic amino acid or a neutral amino acid is replaced by another neutral amino acid, or polar amino acid is replaced by another polar amino acid.

The antibody of the invention may preferably be a mouse antibody, more preferably a mouse monoclonal antibody.

The antibody of the invention is preferably an IgG antibody, more preferably an IgG monoclonal antibody.

Another object of the invention is a method for detecting and quantifying protective antibodies against SARS Cov-2 Spike protein in a sample, comprising the steps of:
(a) incubating the sample with a solid phase coated with the recombinant Spike RBD protein to bind antibodies from the sample to the recombinant Spike RBD protein;
(b) subsequently incubating the sample from step (a) with a conjugate of the antibody of the present invention with a detection tag to bind the conjugate to the remaining unoccupied epitopes on the recombinant Spike RBD protein,
(c) washing out the unbound conjugate, and
(d) detecting a signal generated by means of the detection tag.

The sample is preferably a sample of serum or plasma or whole blood, in particular a sample of human serum or human plasma or human whole blood.

The solid phase may include polystyrene vessels, polyethylene vessels, polypropylene vessels, coated vessels, ELISA vessels, beads, magnetic beads, affinity chromatography resins, nitrocellulose membranes, or PVDF (polyvinylidene fluoride) membranes.

Vessels may include tubes, wells, multiwell plates, or microtitre plates.

Recombinant Spike RBD protein (e.g., amino acids 332 - 529, based on the sequence UniProtKB - PODTC2) is commercially available or may be produced by known techniques. The recombinant Spike RBD protein may be tagged, e.g., with Flag, Strep, His, or biotin tags. The recombinant Spike RBD protein may for example have the sequence SEQ ID NO. 10, as shown in the examples.

The detection tag used for antibody labeling may be enzymatic, fluorescent, chemiluminescent, electrochemiluminescent or radioactive. Chemiluminescence or electrochemiluminescence are often used in fully automated random access systems, while in semi-automated ELISA systems, the enzymatic labeling with the endpoint color detection is preferred.

The enzymatic tag is typically selected from horseradish peroxidase - HRP and alkaline phosphatase - AP. Enzymatic detection is usually carried out by an enzymatic reaction with a substrate that changes color upon the enzymatic reaction. Such substrates may include o-phenylenediamine dihydrochloride (OPD), 3,3',5,5'-tetramethylbenzidine (TMB), 2,2'-azinobis [3-ethylbenzothiazoline-6-sulfonic acid]-diammonium salt (ABTS), or disodium salt of *p-*nitrophenyl phosphate (PNPP). OPD, TMB, and ABTS react with HRP, while PNPP reacts with AP.

In some embodiments, the detection tag is horseradish peroxidase, and the detection step (d) is carried out by detecting a colorimetric signal generated by the horseradish peroxidase using a chromogenic substrate (e.g., 4-chloro-1-naphthol, 3,3'-diaminobenzidine, or 3,3',5,5'-tetramethylbenzidine).

Preferably, the method as described herein has a format of competitive ELISA assay.

The monoclonal antibody of the present invention possesses a unique combination of properties. It recognizes a conformational epitope of Spike RBD protein, inhibits Spike RBD - ACE2 binding, and inhibits viral infection in VNT). More importantly, the inhibition of the viral infection was observed for the wild-type SARS Cov-2 (variant B.1) as well as for the variant of concern (B.1.1.7.) of SARS CoV-2.

The competitive test as described herein utilizes the unique combination of properties of the monoclonal antibody of the present invention to enable quantitative detection of potentially neutralizing antibodies. Moreover, this setting enables to get information about the patient's antibodies protectivity (neutralizing ability) against the SARS CoV-2 virus without demanding laboratory biosafety level 2. The test also offers a much faster quantitative readout (in hours) when compared with the VNTs (in days). Moreover, the predilution of the serum or plasma tested is not necessary.

### Brief Description of the Drawings

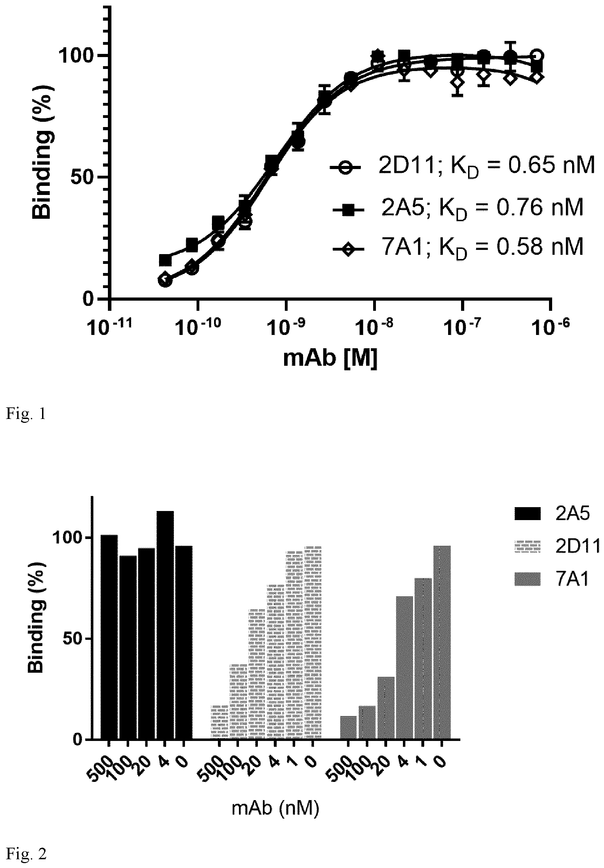

Fig. 1 indicates the affinity of the anti-RBD mAbs produced according to the examples determined by direct ELISA. The x axis displays molar antibody concentrations, and the y axis the percentage of maximum signal in ELISA.
Fig. 2 shows inhibition of Spike RBD - ACE2 interactions by three different monoclonal antibodies (2A5, 2D11, 7A1) recognizing conformational epitopes located on Spike RBD protein. Data for the six different monoclonal antibody concentrations are shown on axis x, and the percentage of antibody maximum binding is displayed on axis y.

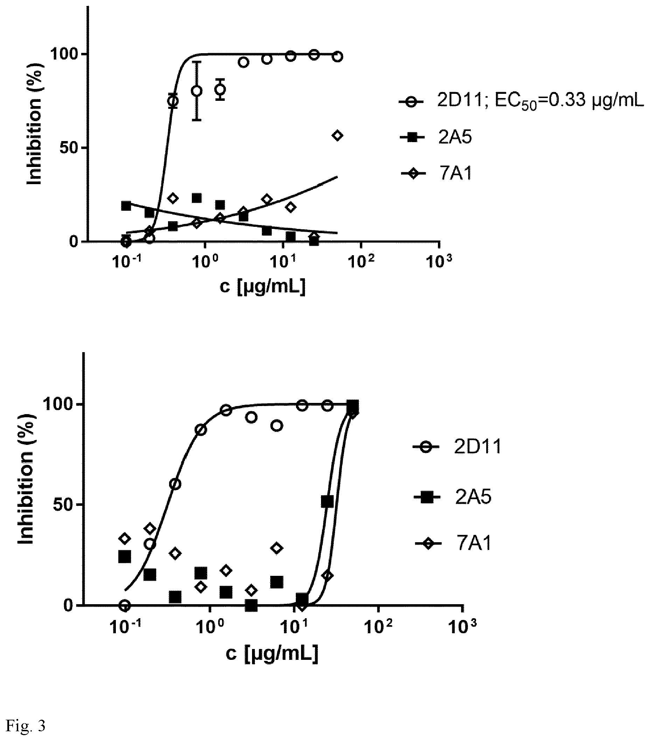

Fig. 3 shows the virus neutralization tests (VNT) results. Three antibody clones recognizing conformational epitopes on Spike RBD were tested with two SARS CoV-2 viruses - the wild-type variant B.1 (top chart) and the variant of concern B.1.1.7 (bottom chart). On axis y, the percentage of viral inhibition is shown. Various concentrations of tested monoclonal antibodies are displayed on the axis x.

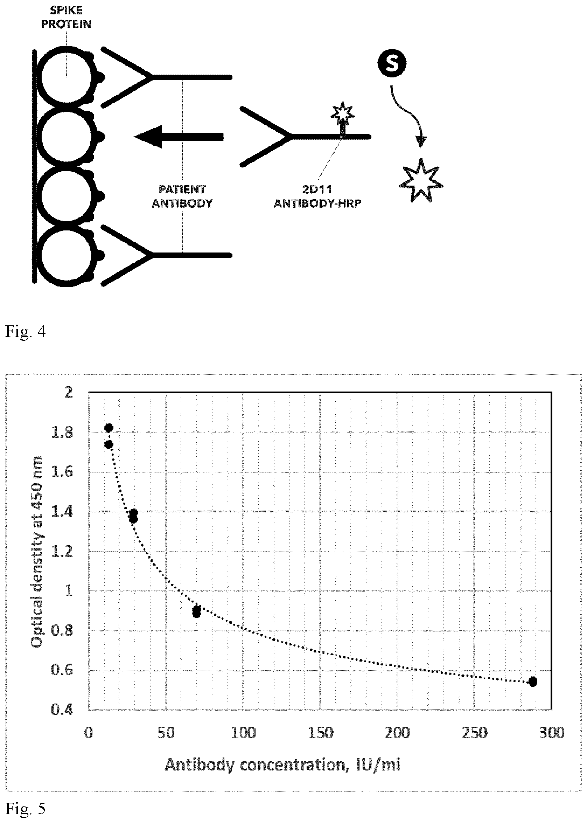

Fig. 4 shows the scheme of competitive ELISA-based SARS CoV-2 assay of the invention as described in the examples. In the first step, the patient antibodies bind to a recombinant Spike RBD protein coated on the polystyrene microplate surface. In the second step, the HRP conjugate of 2D11 monoclonal antibody binds to the remaining accessible RBD epitopes. Thus, the higher is the level of the patient's antibodies blocking the 2D11 epitope, the lower is the colorimetric signal generated.
Fig. 5 shows an example of a calibration curve of the assay of the invention as described in the examples and indicates a dependence of optical density (axis y) response on the concentration of monoclonal antibody 2D11 (axis x) in calibration solutions related to International Standard Anti-SARS CoV-2 code 20/136.

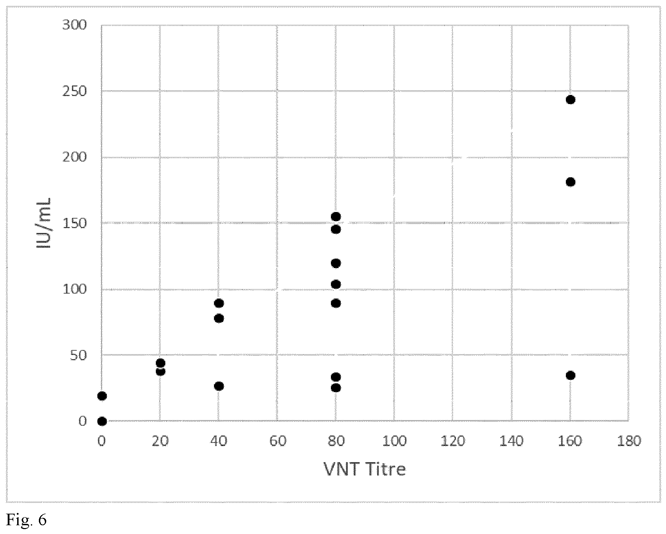

Fig. 6 shows a correlation of the VNT (titer on x axis) with SARS CoV-2 assay of the invention as described in the examples (y axis shows protective antibody concentration in IU/mL) in 27 patients serum samples.

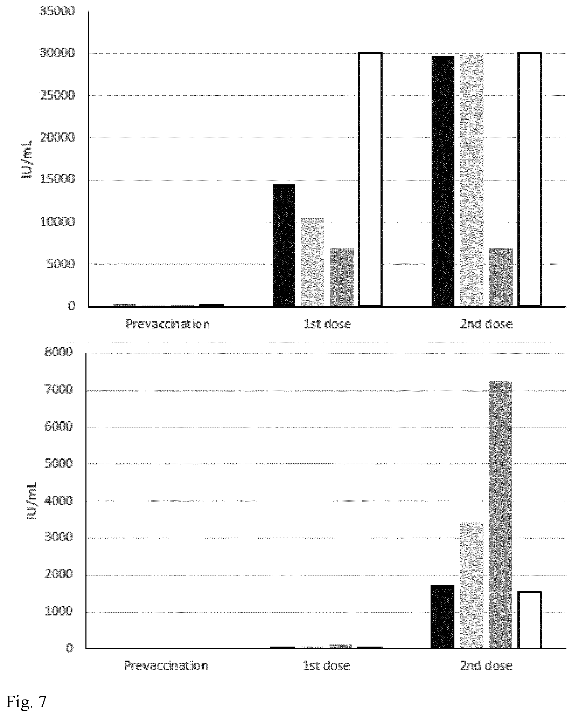

Fig. 7 demonstrates the immune response of four individual patients to vaccination by Pfizer-BioNtech Comirnaty vaccine. The y axis displays the values in IU/mL obtained by the SARS CoV-2 assay of the invention as described in the examples. The immune response of four Covid 19 positive (upper chart) and four Covid 19 naive (bottom chart) patients is shown. Each column represents a sample of one patient.

### Examples

### Example 1: Preparation of recombinant proteins

The recombinant full-length Spike protein (amino acids 19 - 1208, based on UniProtKB - PODTC2, SEQ ID NO. 9) was expressed as a secreted protein with the N-terminal Strep-tag and the C-terminal Avi-tag in HEK293 cells. The Spike sequence is based on sequence available in UniProtKB - PODTC2 with stabilizing mutations and the C-terminal fibrin trimerization motif as described in DOI: 10.1126/science.abb2507.

Full-length Spike protein, SEQ ID NO. 9:

AAQPARRARRTKLGTELGSGGGSAWSHPQFEKGGGSGGGSGGSAWSHPQFEKGGGS
LEVLFQGPAAAMTTRTQLPPAYTNSFTRGVYYPDKVFRSSVLHSTQDLFLPFFSNVT
WFHAIHVSGTNGTKRFDNPVLPFNDGVYFASTEKSNIIRGWIFGTTLDSKTQSLLIVNN

ATNVVIKVCEFQFCNDPFLGVYYHKNNKSWMESEFRVYSSANNCTFEYVSQPFLMD
LEGKQGNFKNLREFVFKNIDGYFKIYSKHTPINLVRDLPQGFSALEPLVDLPIGINITRF
QTLLALHRSYLTPGDSSSGWTAGAAAYYVGYLQPRTFLLKYNENGTITDAVDCALD
PLSETKCTLKSFTVEKGIYQTSNFRVQPTESIVRFPNITNLCPFGEVFNATRFASVYAW
NRKRISNCVADYSVLYNSASFSTFKCYGVSPTKLNDLCFTNVYADSFVIRGDEVRQIA
PGQTGKIADYNYKLPDDFTGCVIAWNSNNLDSKVGGNYNYLYRLFRKSNLKPFERDI
STEIYQAGSTPCNGVEGFNCYFPLQSYGFQPTNGVGYQPYRVVVLSFELLHAPATVC
GPKKSTNLVKNKCVNFNFNGLTGTGVLTESNKKFLPFQQFGRDIADTTDAVRDPQTL
EILDITPCSFGGVSVITPGTNTSNQVAVLYQDVNCTEVPVAIHADQLTPTWRVYSTGS
NVFQTRAGCLIGAEHVNNSYECDIPIGAGICASYQTQTNSPGSASSVASQSIIAYTMSL
GAENSVAYSNNSIAIPTNFTISVTTEILPVSMTKTSVDCTMYICGDSTECSNLLLQYGSF
CTQLNRALTGIAVEQDKNTQEVFAQVKQIYKTPPIKDFGGFNFSQILPDPSKPSKRSFIE
DLLFNKVTLADAGFIKQYGDCLGDIAARDLICAQKFNGLTVLPPLLTDEMIAQYTSAL
LAGTITSGWTFGAGAALQIPFAMQMAYRFNGIGVTQNVLYENQKLIANQFNSAIGKI
QDSLSSTASALGKLQDVVNQNAQALNTLVKQLSSNFGAISSVLNDILSRLDPPEAEVQ
IDRLITGRLQSLQTYVTQQLIRAAEIRASANLAATKMSECVLGQSKRVDFCGKGYHL
MSFPQSAPHGVVFLHVTYVPAQEKNFTTAPAICHDGKAHFPREGVFVSNGTHWFVT
QRNFYEPQIITTDNTFVSGNCDVVIGIVNNTVYDPLQPELDSFKEELDKYFKNHTSPDV
DLGDISGINASVVNIQKEIDRLNEVAKNLNESLIDLQELGKYEQGSGYIPEAPRDGQA
YVRKDGEWVLLSTFLGGAPLEGGGGLNDIFEAQKIEWHE

Recombinant Spike RBD (amino acids 332 - 529, SEQ ID NO. 10) was expressed with the N-terminal Strep-tag and C-terminal Avi-tag in HEK293 cells. Cells were grown in FreeStyle F17 Expression Medium (Gibco, Thermo Fisher Scientific, Waltham, MA, USA) supplemented with 0.1% Pluronic F-68 (Invitrogen, Carlsbad, CA, USA) and 2 mM L-glutamine at 110 rpm under a humidified 5% CO2 atmosphere at 37°C.

Spike RBD domain, SEQ ID NO. 10:

AAQPARRARRTKLGTELGSGGGSAWSHPQFEKGGGSGGGSGGSAWSHPQFEKGGGS
LEVLFQGPAAAMITNLCPFGEVFNATRFASVYAWNRKRISNCVADYSVLYNSASFST
FKCYGVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFNCYFP

LQSYGFQPTNGVGYQPYRVVVLSFELLHAPATVCGPKKGAPLEGGGGLNDIFEAQKI
EWHE

Proteins were heterologously expressed by polyethyleneimine-mediated transfection, and transiently transfected cells were harvested three days post-transfection. Conditioned medium was concentrated and recombinant proteins purified using Streptactin affinity chromatography according to standard protocols. The biotinylated Spike RBD protein was prepared as a fusion with the C-terminal Avi biotinylation sequence, biotinylated in vitro by BirA ligase, and purified by gel exclusion chromatography.

### Example 2: Preparation and selection of monoclonal antibodies

The purified full-length Spike protein with the N-terminal tag cleaved off by the C3 protease was used for the immunization of mice. Eleven unique hybridoma clones expressing IgG monoclonal antibodies were obtained by a standard hybridoma protocol (DOI: 10.1002/pros.23311). They were purified by protein G affinity chromatography and tested for immunoreactivity against the Spike RBD protein fragment by direct ELISA method. A 384-well MaxiSorp plate was coated with streptavidin and loaded with the biotinylated Spike RBD protein. The antibodies were applied in 2-fold dilution series, and binding was detected with a goat anti-mouse secondary antibody conjugated to horseradish peroxidase (Bio-Rad; 1:10,000 dilution). Signals were developed using the 3,3',5,5'-tetramethylbenzidine substrate (Sigma) according to the manufacturer's protocol. Baseline-corrected data were analyzed with Prism 5 software (Fig. 1). Based on their high affinity and specificity for RBD, the 2D11, 7A1, and 2A5 antibody clones were selected from the set of prepared antibodies for further testing. The 2D11 clone is an IgG antibody having the CDRs with sequences SEQ ID NO. 3-8, the VHC with sequence SEQ ID NO. 1, and the VLC with sequence SEQ ID NO. 2.

The three selected clones were further evaluated in two functional tests: inhibition of Spike RBD - ACE2 binding and inhibition of virus infectivity in the VNT. To determine the inhibition of Spike RBD-ACE2 binding, HEK293 cells overexpressing ACE2 (prepared by transfection of HEK 293T cells by full length ACE2 with N-terminal Flag^{®}; selected using Hygromycin antibiotic and prepared clones screened for ACE2 expression using flow cytometry by anti-flag^{®} M2 antibody) were incubated with the mixture of the biotinylated Spike RBD protein (2 nM) and varying concentrations of the antibodies tested. The RBD-ACE2 binding at the cell surface was visualized by flow cytometry analysis using Streptavidin-PE conjugate (Fig. 2.). The graph presents the four concentrations of the tested antibodies onaxis x and the percentage of RBD-ACE2 maximum binding on axis y. The data clearly demonstrate that only the clones 2D11 and 7A1 effectively inhibit the binding of Spike RBD to ACE2 receptor on the cell surface from the three anti-RBD antibody clones tested.

The three anti-RBD antibody clones selected were also tested for the ability to inhibit viral infection in the VNT with two variants of the SARS CoV-2 - wild type (B.1) and the variant of concern (B.1.1.7) (Fig. 3). Two-fold serial dilutions of the monoclonal antibodies were mixed with the SARS-CoV-2 and incubated for 1 hour at room temperature. Then, the mixtures were used to infect VERO E6 cells (MOI = 0.4) for 72 hours in a CO₂ incubator set to 37°C. After incubation, an immunofluorescence (IF) assay was performed. The cultivation medium was washed out. Cells were fixed using 4% paraformaldehyde (PFA). The cell membranes were perforated with 0.2% Triton X-100, and the presence of the SARS-CoV-2 was demonstrated by binding of mouse anti-SARS-CoV-2 antibody (mouse monoclonal nucleoprotein IgG, ProSci, CA, USA) followed by anti-mouse antibody conjugated with Cy3 fluorophore and the resulting signal was analyzed in a fluorescent microscope.

Two of the three antibodies recognizing epitopes on RBD showed minimal efficacy in preventing cell infections in the VNT.

We concluded that not all the antibodies raised against the SARS CoV-2 Spike RBD could provide sufficient protection against the virus, which is a major drawback of all total binding antibody tests. However, the selected 2D11 antibody (which is the antibody of the invention, having the VHC sequence SEQ ID NO. 1 and the VLC sequence SEQ ID NO. 2) overcomes this problem as it directly inhibits the RBD-ACE2 interactions and is able to neutralize the viral infection of susceptible host cells. Due to these properties, the 2D11 monoclonal antibody clone can be used for a quantitative assessment of neutralizing antibodies against the SARS CoV-2 Spike RBD in patient specimens.

### Example 3: Preparation of conjugate of 2D11 antibody with horseradish peroxidase (HRP)

Antibody 2D11 in the quantity of 1 mg was conjugated with 5 mg of Horseradish peroxidase (HRP) using the glutaraldehyde method. First, HRP is activated by 1.25% glutaraldehyde solution in phosphate buffer pH 6.8 for 14 hours. After size exclusion chromatography (Sephadex 25) activated HRP is mixed with IgG in 0.15 M NaCl solution and carbonate-bicarbonate buffer pH 9.5, 5% of the total volume is added. The reaction is stopped after 14 hours by the addition of 0.2 M Lysine solution at 5% of total volume. Superdex HR 200 column was used for the final separation of the IgG-HRP conjugate.

### Example 4: ELISA-based SARS CoV-2 assay of the invention

The 96 well microplates (Greiner) were coated with streptavidin at a coating concentration of 10 ug/mL, washed, and incubated with the biotinylated Spike RBD protein at a concentration of 100 ng/mL. The coated microplates were incubated with 25 microlitres (uL) of patient serum and 150 uL of citrate buffer for 60 min at room temperature and under shaking. Antibodies from a patient serum samples bind to the Spike RBD protein, and after washing, the added 2D 11 monoclonal antibody conjugated to HRP binds to the remaining unoccupied 2D11 binding sites on Spike RBD. After 60 min of incubation at room temperature, the unbound 2D11-HRP conjugate is washed out, and the colorimetric signal is developed by adding TMB. After 10 minutes, the color reaction is stopped, and the resulting OD is analyzed at 450 nm. A set of calibrators with pre-determined concentrations of pure monoclonal antibody 2D11 (without the HRP) is used to construct the calibration curve; The WHO IS anti-SARS Cov-2 standard is used for the value assessment of this calibration set (Fig. 5).

The assay of the invention is the competitive assay, so the higher the content of anti-RBD human antibodies in the sample that blocks the 2D11 binding site on Spike RBD, the lower the colorimetric signal generated.

The SARS CoV-2 assay as described herein was tested to demonstrate the correlation of this assay with the VNT. The 27 patient serum samples were tested in parallel by the SARS CoV-2 assay of the invention and the VNT. The samples were tested by a certified VNT method in an accredited lab at Public Health Institute Ostrava (doi: 10.3390/diagnostics11040593.), and the result is shown in Fig.6.

We also collected and analyzed samples from patients subjected to the SARS CoV-2 vaccination by the Pfizer-BioNtech Comirnaty vaccine. The serum samples were collected before the first vaccination, 20-21 days after the first vaccination dose administration, and 30 days after the second vaccination dose. The results are shown in Fig. 7.

The SARS CoV-2 assay of the invention measures only the antibodies competing with the 2D11 monoclonal antibody for the Spike RBD epitope. Since the 2D 11 epitope has been demonstrated to be critical for the virus recognition and binding to ACE2 and also for the infectivity of the SARS CoV-2 in the VNT, the antibodies in the patient serum (plasma, blood) competing for the binding site with the 2D11 monoclonal antibody are potentially neutralizing antibodies.

### Industrial applicability

The assay of the present invention can be used as a stand-alone test for the assessment of protective antibodies level after Covid 19 vaccination and/or SARS CoV-2 infection or can serve as a confirmatory test for the grey zone results of standard (total) anti-Spike antibody assay.

The assay is designed for long-term monitoring of the SARS CoV-2 neutralizing antibody level in health care professionals and for monitoring the patients with the increased risk of severe infection.

## Claims

1. An antibody binding to RBD of Spike protein of the SARS-CoV-2, wherein the complementarity determining regions of the variable heavy chain region have sequences:
TSGMGVS (SEQ ID NO. 3),
HIYWDDDERYNPSLKS (SEQ ID NO. 4), and
RSLYDYDGWFAY (SEQ ID NO. 5);
and the complementarity determining regions of the variable light chain region have sequences:
KASQNVDTTVA (SEQ ID NO. 6),
SASSRYS (SEQ ID NO. 7), and
QQYNNYPLT (SEQ ID NO. 8).

2. The antibody according to claim 1, wherein the variable heavy chain region has a sequence with at least 90% identity with the sequence EVQLRESGPGILQPSQTLSLTCSFSGFSLSTSGMGVSWIRQPSGKGLEWLAHIYWDDD ERYNPSLKSRLTISKDVSSNOVFLKIISVDTADTATYYCARRSLYDYDGWFAYWGOG TLVTVSA (SEQ ID NO. 1), wherein the underlined portions corresponding to the complementarity determining regions are conserved.

3. The antibody, according to claim 1, wherein the variable heavy chain region has a sequence differing by no more than 5, and more preferably by no more than 3, amino acid substitutions from the sequence EVQLRESGPGILQPSQTLSLTCSFSGFSLSTSGMGVSWIRQPSGKGLEWLAHIYWDDD ERYNPSLKSRLTISKDVSSNOVFLKIISVDTADTATYYCARRSLYDYDGWFAYWGOG TLVTVSA (SEQ ID NO. 1), wherein the amino acid substitutions are not in the underlined portions corresponding to the complementarity determining regions.

4. The antibody according to any one of claims 1 to 3, wherein the variable light chain region has a sequence with at least 90% identity with the sequence DIVMTQSQKFMSTSVGDRVSVTCKASQNVDTTVAWYOOKPGOSPKALIYSASSRYS GVPDRFTGSGSGTDFTLTISNVQSEDLADYFCQQYNNYPLTFGAGTKLEIKRA (SEQ ID NO. 2), wherein the underlined portions corresponding to the complementarity determining regions are conserved.

5. The antibody according to any one of claims 1 to 3, wherein the variable light chain region has a sequence differing by no more than 5, and more preferably by no more than 3, amino acid substitutions from the sequence DIVMTQSQKFMSTSVGDRVSVTCKASQNVDTTVAWYQQKPGQSPKALIYSASSRYS GVPDRFTGSGSGTDFTLTISNVQSEDLADYFCQQYNNYPLTFGAGTKLEIKRA (SEQ ID NO. 2), wherein the amino acid substitutions are not in the underlined portions corresponding to the complementarity determining regions.

6. The antibody, according to any one of claims 1 to 5, wherein the antibody is a mouse monoclonal antibody.

7. The antibody, according to any one of claims 1 to 6, wherein the antibody is an IgG monoclonal antibody.

8. A method for detecting and quantifying protective antibodies against the SARS CoV-2 Spike protein in a sample, comprising the steps of:
(a) incubating the sample with a solid phase coated with the recombinant Spike RBD protein to bind antibodies from the sample to the recombinant Spike RBD protein;
(b) subsequently incubating the sample from step (a) with a conjugate of the antibody according to any one of claims 1 to 7 with a detection tag, to bind the conjugate to the remaining unoccupied epitopes on the recombinant Spike RBD protein,
(c) washing out the unbound conjugate, and
(d) detecting a signal generated by means of the detection tag.

9. The method according to claim 8, wherein the sample is a sample of serum or plasma or whole blood.

10. The method according to claim 8 or 9, wherein the solid phase is selected from polystyrene vessels, polypropylene vessels, ELISA vessels, beads, magnetic beads, affinity chromatography resins, nitrocellulose membranes, or PVDF (polyvinylidene fluoride) membranes.

11. The method according to any one of claims 8 to 10, wherein the detection tag in the antibody conjugate is selected from enzymatic, fluorescent, chemiluminescent, electrochemiluminescent and radioactive tags.

12. The method according to any one of claims 8 to 11, wherein the detection tag is horseradish peroxidase and the detection step (d) is carried out by detecting a colorimetric signal generated by the horseradish peroxidase using a chromogenic substrate, preferably the chromogenic substrate is selected from 4-chloro-1-naphthol, 3,3'-diaminobenzidine, or 3,3',5,5'-tetramethylbenzidine.
